(19) Europäisches Patentamt
European Patent Office
Office européen des brevets

(11) Veröffentlichungsnummer: **0 086 392**
**B1**

# (12) EUROPÄISCHE PATENTSCHRIFT

(45) Veröffentlichungstag der Patentschrift: **21.08.85**

(51) Int. Cl.⁴: **A 01 N 43/64,** A 01 N 43/40, A 01 N 39/02

(21) Anmeldenummer: **83100897.4**

(22) Anmeldetag: **01.02.83**

(54) **Herbizide Mittel enthaltend Metamitron in Kombination mit bestimmten Phenoxypropionsäureestern.**

(30) Priorität: **12.02.82 DE 3205103**

(43) Veröffentlichungstag der Anmeldung: **24.08.83 Patentblatt 83/34**

(45) Bekanntmachung des Hinweises auf die Patenterteilung: **21.08.85 Patentblatt 85/34**

(84) Benannte Vertragsstaaten: **AT BE CH DE FR GB IT LI NL SE**

(56) Entgegenhaltungen:
**EP - A - 0 004 414**
**EP - A - 0 043 800**
**DE - A - 2 657 583**
**FR - A - 2 321 239**

(73) Patentinhaber: **BAYER AG, Konzernverwaltung RP Patentabteilung, D-5090 Leverkusen 1 Bayerwerk (DE)**

(72) Erfinder: **Schmidt, Robert R., Dr., Im Waldwinkel 110, D-5060 Bergisch-Gladbach 2 (DE)**

**EP 0 086 392 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft herbizide, synergistische Wirkstoffkombinationen, die aus Metamitron einerseits und bestimmten Phenoxypropionsäureestern andererseits bestehen und mit besonderem Vorteil zur selektiven Unkrautbekämpfung in Rübenkulturen verwendet werden können.

Es ist bereits bekannt geworden, daß 4-Amino-3-methyl-6-phenyl-1,2,4-triazin-5 (4 H)- on (common name: Metamitron) als selektives Herbizid in Rübenkulturen eingesetzt werden kann (vgl. DE-OS 2 224 161); Metamitron wirkt jedoch nur schwach gegen hirseartige Ungräser.

Es wurde nun überraschend gefunden, daß Wirkstoffkombinationen bestehend aus

(1) 4-Amino-3-methyl-6-phenyl-1,2,4-triazin-5 (4 H)-on (Metamitron) der Formel I

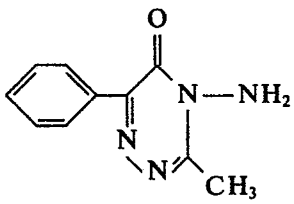

(I)

und

(2) mindestens einem substituierten Phenoxypropionsäureester der allgemeinen Formel II

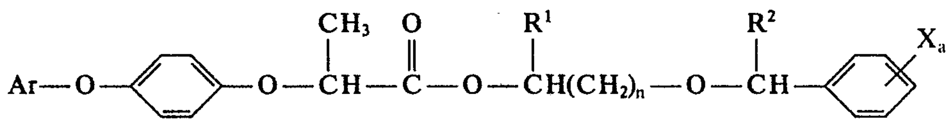

(II)

worin

$R^1$ für Wasserstoff oder Methyl steht,
$R^2$ für Wasserstoff oder Methyl steht,
n für 1 oder 2 steht,
X für Wasserstoff, Methyl, Methoxy, Halogen und/oder Nitro steht,
a für 1 oder 2 steht,
Ar für den Rest

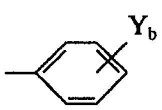 oder 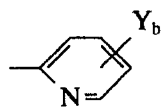

steht,
Y für $C_{1-4}$-Alkyl, Halogen, Trifluormethyl, Nitro und/oder Cyano steht und
b für 1, 2 oder 3 steht,

eine besonders hohe herbizide Wirksamkeit aufweisen.

Besonders bevorzugte Wirkstoffe gemäß Formel (II) sind die folgenden Verbindungen:

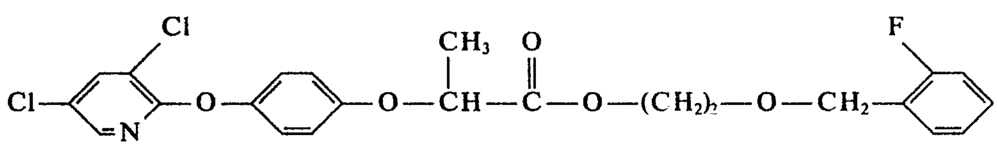

(II-1)

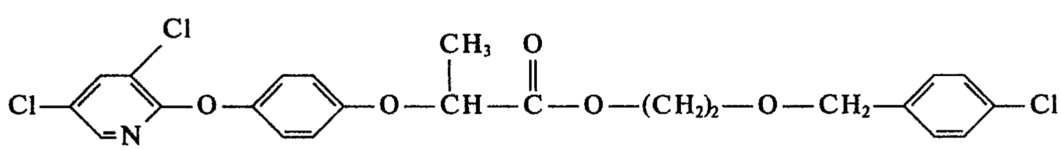

(II-2)

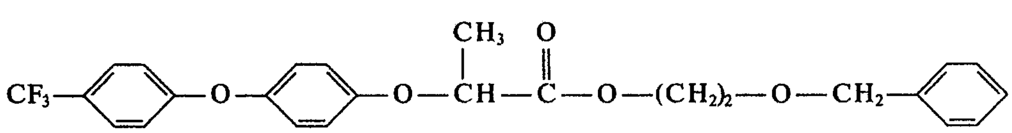

(II-3)

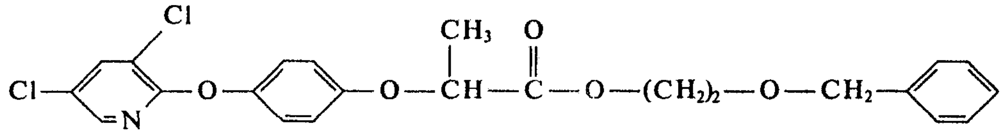 (II-4)

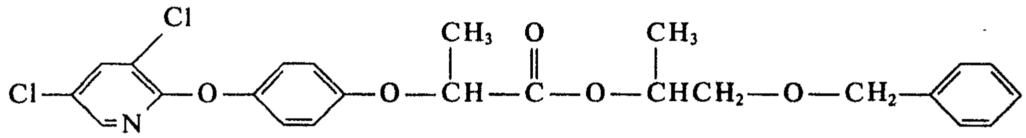 (II-5)

Überraschenderweise ist die herbizide Wirksamkeit der erfindungsgemäßen Wirkstoffkombinationen wesentlich höher als die Summe der Wirkungen der einzelnen Wirkstoffe. Es liegt somit ein nicht vorhersehbarer synergistischer Effekt vor und nicht nur eine Wirkungsergänzung. Die neuen Wirkstoffkombinationen sind ebenso wie Metamitron (I) in Rübenkulturen sehr gut verträglich, wobei die neuen Wirkstoffkombinationen auch die » Problemunkräuter« — hirseartiger Ungräser und Flughafer — hervorragend bekämpfen. Die neuen Wirkstoffkombinationen stellen somit eine wertvolle Bereicherung der Rübenherbizide dar.

Als Unkräuter, die im allgemeinen als Verunreinigung in Rübenkulturen auftreten und durch die erfindungsgemäßen Wirkstoffkombinationen sicher bekämpft werden können, seien beispielsweise genannt:

dikotyle Unkräuter der Gattungen:
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea; sowie

monokotyle Unkräuter der Gattungen:
Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Elocharis, Scirpus, Paspalum, Ischaemum, Sphenoclea, Dactyloctenium, Agrostis, Alopecurus, Apera.

Die Verwendung der erfindungsgemäßen Wirkstoffkombinationen ist jedoch keineswegs auf diese Gattungen beschränkt, sondern erstreckt sich in gleicher Weise auch auf andere Pflanzen.

Die erfindungsgemäßen Wirkstoffkombinationen weisen, wie bereits angegeben, bei sehr guter Verträglichkeit gegenüber Rübenkulturen eine hervorragende Wirkung gegen Unkräuter und Ungräser auf, insbesondere gegen hirseartige Ungräser. Ihr Einsatz als selektive Rübenherbizide ist daher besonders bevorzugt.

Der synergistische Effekt der erfindungsgemäßen Wirkstoffkombinationen ist bei bestimmten Konzentrationsverhältnissen besonders stark ausgeprägt. Jedoch können die Gewischtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001—20 Gewichtsteile, vorzugsweise 0,01—10 Gewichtsteile Wirkstoff aus der Wirkstoffgruppe (II).

Die erfindungsgemäßen Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in üblicher Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykolether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige körnige oder latexförmige Polymere verwendet werden, wie Gummiarabicum, Polyvinylalkohol, Polyvinylacetat.

In den Formulierungen können als weitere Zusätze Farbstoffe wie anorganische Pigmente, zum Beispiel Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffkombination, vorzugsweise zwischen 0,5 und 90%.

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch als Einzelformulierungen bei der Anwendung gemischt, d. h. in Form von Tankmischungen zur Anwendung gebracht werden.

Die neuen Wirkstoffkombinationen können als solche oder in ihren Formulierungen weiterhin auch in Mischung mit anderen bekannten Rübenherbiziden Verwendung finden, wobei wiederum Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich. Für bestimmte Anwendungszwecke kann es ferner vorteilhaft sein, in die Formulierungen als weitere Zusatzstoffe pflanzenverträgliche mineralische oder vegetabilische Öle (z. B. das Handelspräparat »Sun Oil 11E«) oder Ammoniumsalze wie z. B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Stäuben oder Streuen.

Die Aufwandmengen der erfindungsgemäßen Wirkstoffkombination können in einem gewissen Bereich variiert werden; sie hängen u. a. vom Wetter und von den Bodenfaktoren ab.

Im allgemeinen liegen die Aufwandmengen zwischen 0,01 und 15 kg Wirkstoffkombination pro ha, vorzugsweise zwischen 0,05 und 10 kg/ha.

Die erfindungsgemäßen Wirkstoffkombinationen können sowohl vor als auch nach dem Auflaufen der Pflanzen appliziert werden. Die Aufwendung wird vorzugsweise nach dem Auflaufen der Pflanzen, also im post-emergence-Verfahren, vorgenommen.

Die gute herbizide Wirkung der neuen Wirkstoffkombinationen geht aus den nachfolgenden Beispielen hervor. Während die einzelnen Wirkstoffe in der herbiziden Wirkung Schwächen aufweisen, zeigen die Kombinationen eine sehr breite Unkrautwirkung, die über eine einfache Wirkungssummierung hinausgeht.

Ein synergistischer Effekt liegt bei Herbiziden immer dann vor, wenn die herbizide Wirkung der Wirkstoffkombination größer ist als die der einzelnen applizierten Wirkstoffe.

Die zu erwartende Wirkung für eine gegebene Kombination zweier Herbizide kann wie folgt berechnet werden (vgl. COLBY, S.R., »Calculating synergistic and antagonistic responses of herbicide combinations«, Weeds 15, Seiten 20–22, 1967):

Wenn

X = % Schädigung durch Herbizid A bei p kg/ha Aufwandmenge und
Y = % Schädigung durch Herbizid B bei q kg/ha Aufwandmenge und
E = die erwartete Schädigung der Herbizide A und B, bei p und q kg/ha Aufwandmenge,

dann ist

$$E = X + Y - \frac{X \cdot Y}{100}.$$

Ist die tatsächliche Schädigung größer als berechnet, so ist die Kombination in ihrer Wirkung überadditiv, d. h. sie zeigt einen synergistischen Effekt.

Aus den nachfolgenden Beispielen geht hervor, daß die gefundene herbizide Wirkung der erfindungsgemäßen Wirkstoffkombinationen bei den Unkräutern größer ist als die berechnete, d. h., daß die neuen Wirkstoffkombinationen synergistisch wirken.

Die erfindungsgemäßen Wirkstoffkombinationen zeigen zum Teil auch eine Nebenwirkung gegen Bodeninsekten sowie eine systemische Wirkung gegen Pyricularia oryzae an Reis, welche auf einer entsprechenden Wirkung der Wirkstoffkomponente (II) beruhen dürfte.

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5–15 cm haben so, daß die in der Tabelle angegebenen Wirkstoffmengen pro Flächeneinheit ausgebracht werden. Die Konzentration der Spritzbrühe wird so gewählt, daß in 2000 l Wasser/ha die in der Tabelle angegebenen Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Wirkstoffe, Aufwandmengen und Resultate gehen aus der nachfolgenden Tabelle A hervor:

Tabelle A

Post-emergence-Test/Gewächshaus

| Wirkstoff bzw. Wirkstoff-kombination**) | Wirkstoff-aufwandmenge kg/ha | Schädigung bzw. Wirkung in % | | | | | | | |
|---|---|---|---|---|---|---|---|---|---|
| | | Zuckerrüben | | Digitaria | | Setaria | | Echinochloa | |
| | | gef.*) | ber.*) | gef.*) | ber.*) | gef.*) | ber.*) | gef.*) | ber.*) |
| bekannt | | | | | | | | | |
| (I) | 2,8 | 0 | | 20 | | 0 | | 0 | |
| (II-1) | 0,125 | 0 | | 40 | | 10 | | 40 | |
| (II-2) | 0,125 | 0 | | 50 | | 10 | | 30 | |
| (II-3) | 0,125 | 0 | | 40 | | 0 | | 45 | |
| (II-4) | 0,125 | 0 | | 40 | | 20 | | 30 | |
| (II-5) | 0,125 | 0 | | 40 | | 0 | | 50 | |
| erfindungsgemäß | | | | | | | | | |
| (I) + (II-1) | 2,8 + 0,125 | 10 | 0 | 90 | 52 | 95 | 10 | 90 | 40 |
| (I) + (II-2) | 2,8 + 0,125 | 0 | 0 | 80 | 60 | 95 | 10 | 85 | 30 |
| (I) + (II-3) | 2,8 + 0,125 | 10 | 0 | 85 | 52 | 95 | 0 | 85 | 45 |
| (I) + (II-4) | 2,8 + 0,125 | 0 | 0 | 80 | 52 | 95 | 20 | 85 | 30 |
| (I) + (II-5) | 2,8 + 0,125 | 10 | 0 | 95 | 52 | 99 | 0 | 85 | 50 |

*) gef. = gefundene Schädigung (in Prozent)
*) ber. = nach der COLBY-Formel berechnete Schädigung (in Prozent)
**) Wirkstoff (I) = Metamitron; die Strukturformeln der übrigen Wirkstoffe sind mit gleicher Nummer unter den Herstellungsbeispielen angegeben.

Der Wirkstoff der Formel (I) ist bekannt (DE-OS 2 224 161 und US-PS 3 847 914). Die Wirkstoffe der Formel (II) sind noch nicht druckschriftlich beschrieben worden; sie sind Gegenstand der JP-Patentanmeldung Nr. Sho 56-97 486 vom 25. 06. 1981 sowie der JP-Patentanmeldung Nr. Sho-1444 778 vom 16. 09. 1981. Die Wirkstoffe der allgemeinen Formel II

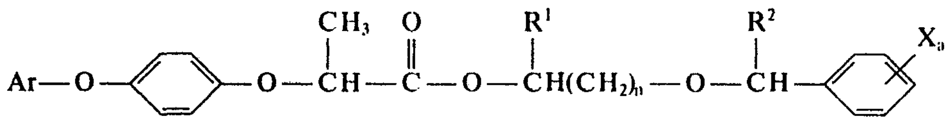

(II)

worin

$R^1$ für Wasserstoff oder Methyl steht,
$R^2$ für Wasserstoff oder Methyl steht,
n für 1 oder 2 steht,
X für Wasserstoff, Methyl, Methoxy, Halogen und/oder Nitro steht,
a für 1 oder 2 steht,
Ar für den Rest

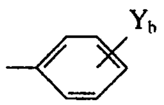

oder

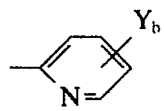

steht,
Y für $C_{1-4}$-Alkyl, Halogen, Trifluormethyl, Nitro und/oder Cyano steht und
b für 1, 2 oder 3 steht,

können hergestellt werden, indem man entweder

(a) eine Verbindung der allgemeinen Formel III

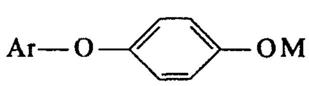

(III)

worin

M für Wasserstoff oder ein Alkalimetall-Atom steht und
Ar die oben angegebene Bedeutung hat,

mit einer Verbindung der allgemeinen Formel IV

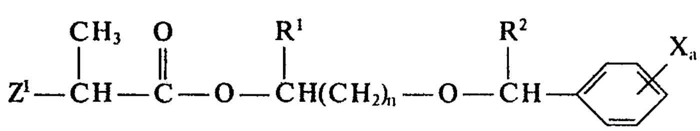

(IV)

worin

$Z^1$ für Halogen steht und
$R^1$, $R^2$, X, n und a die oben angegebene Bedeutung haben,

umsetzt oder indem man

(b) eine Verbindung der allgemeinen Formel V

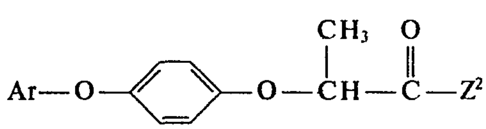

(V)

worin

$Z^2$ für Hydroxy oder Halogen steht und
Ar die oben angegebene Bedeutung hat,

mit einer Verbindung der allgemeinen Formel VI

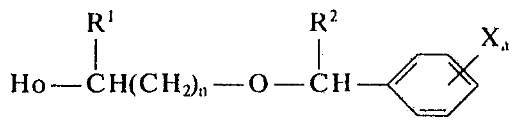

(VI)

worin

$R^1$, $R^2$, X, n und a die oben angegebene Bedeutung haben,

umsetzt.

Verwendet man nach Verfahrensweg (a) beispielsweise 4-(3,5-Dichlor-2-pyridyloxy)-phenol-Natriumsalz und 2-Brompropionsäure-[2-(2-fluorbenzyloxy)]-ethylester als Ausgangsstoffe, so läßt sich der Reaktionsablauf durch das folgende Formelschema wiedergeben:

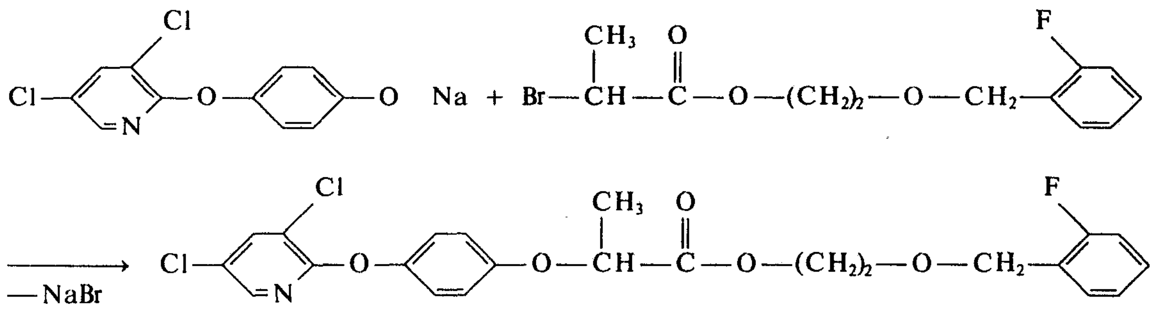


Als bevorzugte Beispiele für Ausgangsstoffe der Formel (III) sind zu nennen:

4-(4-Trifluormethylphenoxy)-phenol,
4-(2-Trifluormethylphenoxy)-phenol,
4-(4-Fluorphenoxy)-phenol,
4-(2,4-Dichlorphenoxy)-phenol,
4-(2-Chlor-4-nitrophenoxy)-phenol,
4-(2-Trifluormethyl-4-chlorphenoxy)-phenol,
4-(4-Trifluormethyl-2-chlorphenoxy)-phenol,
4-(3,5-Dichlor-2-pyridyloxy)-phenol,
4-(5-Nitro-2-pyridyloxy)-phenol,
4-(4-Nitrophenoxy)-phenol
4-(4-Brom-2-chlorphenoxy)-phenol,
4-(4-Trifluormethyl-2-nitrophenoxy)-phenol,
4-(2,6-Dichlor-4-trifluormethylphenoxy)-phenol,
4-(2-Cyano-4-trifluormethylphenoxy)-phenol,
4-(2-Chlor-4-cyanophenoxy)-phenol,
4-(3-Chlor-5-nitro-2-pyridyloxy)-phenol,
4-(5-Trifluormethyl-2-pyridyloxy)-phenol,
4-(3-Chlor-5-trifluormethyl-2-pyridyloxy)-phenol,
4-(5-Brom-3-chlor-2-pyridyloxy)-phenol und
4-(4-Chlor-2-methylphenoxy)-phenol;

es können auch die Alkalimetallsalze, vorzugsweise die Natrium- und Kaliumsalze, der vorgenannten Phenole eingesetzt werden.

Als bevorzugte Ausgangsstoffe der Formel (IV) sind zu nennen:

2-Benzyloxyethyl-2-chlor-(oder -brom)-propionat,
3-Benzyloxypropyl-2-chlor(oder -brom)-propionat,
1-Methyl-2-benzyloxyethyl-2-chlor-(oder -brom)-propionat,
2-$\alpha$-Methylbenzyloxyethyl-2-chlor-(oder -brom)-propionat,
2-(2-Fluorbenzyloxy)-ethyl-2-chlor-(oder -brom)-propionat,
2-(2-Chlorbenzyloxy)-ethyl-2-chlor-(oder -brom)-propionat,
2-(4-Chlorbenzyloxy)-ethyl-2-chlor-(oder -brom)-propionat,
2-(2,4-Dichlorbenzyloxy)-ethyl-2-chlor-(oder -brom)-propionat,
2-(3,4-Dichlorbenzyloxy)-ethyl-2-chlor-(oder -brom)-propionat,
2-(2,6-Dichlorbenzyloxy)-ethyl-2-chlor-(oder -brom)-propionat,
2-(4-Fluorbenzyloxy)-ethyl-2-chlor-(oder brom)-propionat,
2-(2-Methylbenzyloxy)-ethyl-2-chlor-(oder -brom)-propionat,

2-(3-Nitrobenzyloxy)-ethyl-2-chlor-(oder -brom)-propionat,
2-(4-Methoxybenzyloxy)-ethyl-2-chlor-(oder -brom)-propionat,
2-(4-Brombenzyloxy)-ethyl-2-chlor-(oder -brom)-propionat,
1-Methyl-2-α-methylbenzyloxyethyl-2-chlor-(oder -brom)-propionat,
2-(3-Chlorbenzyloxy)-ethyl-2-chlor-(oder -brom)-propionat,
2-(2-Brombenzyloxy)-ethyl-2-chlor-(oder -brom)-propionat,
2-(3-Fluorbenzyloxy)-ethyl-2-chlor-(oder -brom)-propionat,
1-Methyl-2-(2-fluorbenzyloxy)-ethyl-2-chlor-(oder -brom)-propionat,
3-(2-Fluorbenzyloxy)-propyl-2-chlor-(oder -brom)-propionat,
2-(2-Nitrobenzyloxy)-ethyl-2-chlor-(oder -brom)-propionat,
2-(4-Nitrobenzyloxy)-ethyl-2-chlor-(oder -brom)-propionat,
2-(4-Methylbenzyloxy)-ethyl-2-chlor-(oder -brom)-propionat,
2-(2-Methoxybenzyloxy)-ethyl-2-chlor-(oder -brom)-propionat und
3-(4-Fluorbenzyloxy)-propyl-2-chlor-(oder -brom)-propionat.

Verwendet man nach Verfahrensweg (b) beispielsweise 2-[4-(5-Trifluormethyl-2-pyridyloxy)-phenoxy]-propionylchlorid und 1-(4-Chlorbenzyloxy)-2-propanol als Ausgangsstoffe, so läßt sich der Reaktionsablauf durch das folgende Formelschema wiedergeben:

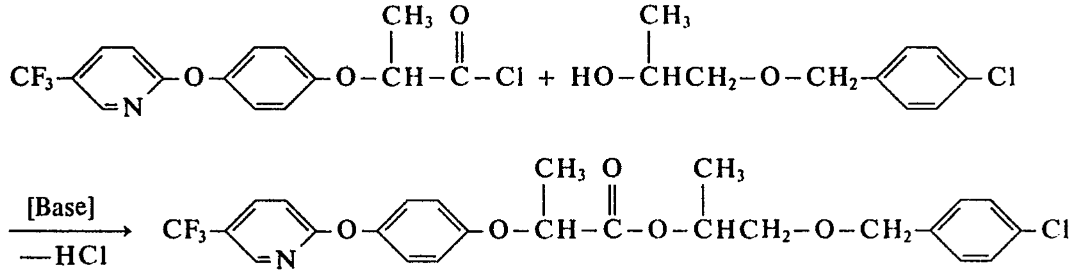


Als bevorzugte Beispiele für Ausgangsstoffe der Formel (V) sind zu nennen:

2-[4-(4-Trifluormethylphenoxy)-phenoxy]-propionyl-chlorid,
2-[4-(2-Trifluormethylphenoxy)-phenoxy]-propionyl-chlorid,
2-[4-(4-Fluorphenoxy)-phenoxy]-propionyl-chlorid,
2-[4-(2,4-Dichlorphenoxy)-phenoxy]propionyl-chlorid,
2-[4-(2-Chlor-4-nitrophenoxy)-phenoxy]-propionyl-chlorid,
2-[4-(2-Trifluormethyl-4-chlorphenoxy)-phenoxy]-propionyl-chlorid,
2-[4-(3,5-Dichlor-2-pyridyloxy)-phenoxy]-propionyl-chlorid,
2-[4-(4-Trifluormethyl-2-chlorphenoxy)-phenoxy]-propionyl-chlorid,
2-[4-(5-Nitro-2-pyridyloxy)-phenoxy]-propionyl-chlorid,
2-[4-(4-Nitrophenoxy)-phenoxy]-propionyl-chlorid,
2-[4-(4-Brom-2-chlorphenoxy)-phenoxy]-propionyl-chlorid,
2-[4-(4-Trifluormethyl-2-nitrophenoxy)-phenoxy]-propionyl-chlorid,
2-[4-(2,6-Dichlor-4-trifluormethylphenoxy)-phenoxy]-propionyl-chlorid,
2-[4-(2-Cyano-4-trifluormethylphenoxy)-phenoxy]-propionyl-chlorid,
2-[4-(2-Chlor-4-cyanophenoxy)-phenoxy]-propionyl-chlorid,
2-[4-(3-Chlor-5-nitro-2-pyridyloxy)-phenoxy]-propionyl-chlorid,
2-[4-(5-Trifluormethyl-2-pyridyloxy)-phenoxy]-propionyl-chlorid,
2-[4-(3-Chlor-5-trifluormethyl-2-pyridyloxy)-phenoxy]-propionyl-chlorid,
2-[4-(5-Brom-3-chlor-2-pyridyloxy)-phenoxy]-propionyl-chlorid und
2-[4-(4-Chlor-2-methylphenoxy)-phenoxy]-propionyl-chlorid;

anstelle der Säurechloride können auch die entsprechenden Säurebromide oder freien Propionsäuren eingesetzt werden.

Als bevorzugte Beispiele für Ausgangsstoffe der Formel (VI) sind zu nennen:

2-Benzyloxyethanol,
3-Benzyloxypropanol,
1-Benzyloxy-2-propanol,
2-α-Methylbenzyloxyethanol,
2-(2-Fluorbenzyloxy)-ethanol,
2-(4-Fluorbenzyloxy)-ethanol,
2-(2-Chlorbenzyloxy)-ethanol,
2-(4-Chlorbenzyloxy)-ethanol,

2-(2,4-Dichlorbenzyloxy)-ethanol,
2-(3,4-Dichlorbenzyloxy)-ethanol,
2-(2,6-Dichlorbenzyloxy)-ethanol,
2-(2-Methylbenzyloxy)-ethanol,
2-(3-Nitrobenzyloxy)-ethanol,
2-(4-Methoxybenzyloxy)-ethanol,
2-(4-Brombenzyloxy)-ethanol,
1-(α-Methylbenzyloxy)-2-propanol,
2-(3-Chlorbenzyloxy)-ethanol,
2-(2-Brombenzyloxy)-ethanol,
2-(3-Fluorbenzyloxy)-ethanol,
1-(2-Fluorbenzyloxy)-2-propanol,
3-(2-Fluorbenzyloxy)-propanol,
2-(2-Nitrobenzyloxy)-ethanol,
2-(4-Nitrobenzyloxy)-ethanol,
2-(4-Methylbenzyloxy)-ethanol,
2-(2-Methoxybenzyloxy)-ethanol und
3-(4-Fluorbenzyloxy)-propanol.

Beide Verfahren werden bevorzugt in Gegenwart eines Verdünnungsmittels durchgeführt. Geeignete Verdünnungsmittel sind Wasser sowie inerte organische Lösungsmittel, z. B. gegebenenfalls chlorierte Kohlenwasserstoffe wie Benzol oder Chloroform, Ether wie Dioxan und Tetrahydrofuran, Nitrile wie Acetonitril, Sulfone und Sulfoxide wie Dimethylsulfoxid bzw. Sulfolan oder tertiäre Basen wie Pyridin.

Beide Verfahren werden bevorzugt in Gegenwart eines Säurebindemittels durchgeführt. Hierfür kommen alle üblicherweise verwendeten Säurebinder in Frage, z. B. Alkalihydroxide, -carbonate, -bicarbonate und -alkoholate, oder tertiäre Amine wie Triethylamin, Diethylanilin oder Pyridin.

Die Reaktionstemperaturen können bei beiden Verfahren in einem größeren Bereich variiert werden. Im allgemeinen arbeitet man bei Temperaturen zwischen −20°C und dem Siedepunkt des jeweiligen Reaktionsgemisches, vorzugsweise zwischen 0°C und 100°C.

Bei der Durchführung der beiden Verfahren werden die Ausgangsstoffe im allgemeinen in etwa äquimolaren Mengen eingesetzt. Säurebindemittel werden in stöchiometrischen oder bis zu dreifach überstöchiometrischen Mengen eingesetzt.

Die Aufarbeitung und Isolierung der Reaktionsprodukte (II) erfolgt in üblicher Weise. Die folgenden Herstellungsbeispiele dienen zur weiteren Erläuterung der beschriebenen Herstellungsverfahren.

## Herstellungsbeispiele

### Beispiel 1

nach Verfahren (a)

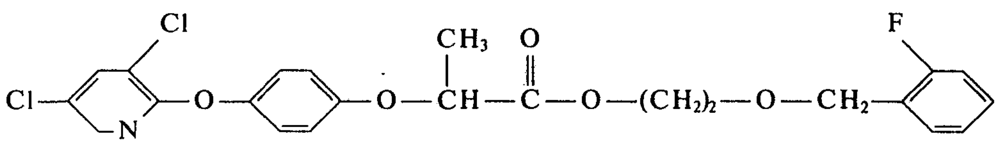
(II-1)

Eine Mischung von 30.6 g 4-(3,5-Dichlor-2-pyridyloxy)-phenol-Natriumsalz und 36 g 2-Brompropionsäure-[2-(2-fluorbenzyloxy)]-ethylester (IV1) in 100 ml Dimethylformamid wurde für 3 Stunden unter Rühren auf 70–80°C erwärmt. Nach Abkühlung auf Raumtemperatur wurde das Reaktionsgemisch in 300 ml Wasser gegossen, dann wurde mit Ether extrahiert; die etherische Phase wurde zunächst mit 1 %-Natronlauge, dann mit Wasser gewaschen und getrocknet. Nach Abdestillieren des Ethers erhält man als Rückstand 38 g des gewünschten 2-[4-(3,5-Dichlor-2-pyridyloxy)-phenoxy]-propionsäure-[2-(2-fluorbenzyloxy)]-ethylesters (II-1); Schmelzpunkt 54–57°C.

### Beispiel 2

nach Verfahren (a)

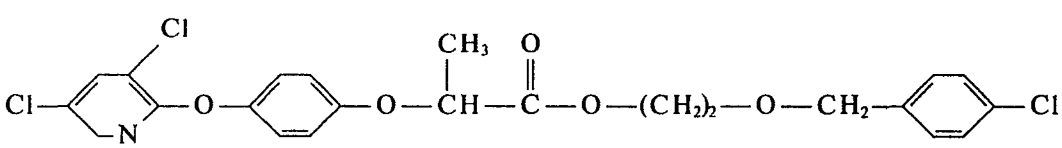
(II-2)

Analog zu Beispiel 1 wurde durch Umsetzung von 4-(3,5-Dichlor-2-pyridyloxy)-phenol-Natriumsalz mit 2-Brompropionsäure-[2-(4-chlorbenzyloxy)]-ethylester (IV-6) die Verbindung 2-[4-(3,5-Dichlor-2-pyridyloxy)-phenoxy]-propionsäure-[2-(4-chlorbenzyloxy)]-ethylester (II-2) hergestellt;
$n_D^{20}$: 1,5803

Beispiel 3

nach Verfahren (a)

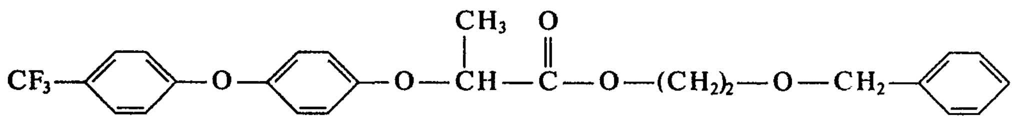
(II-3)

Eine Mischung von 25,4 g 4-(4-Trifluormethyl-phenoxy)-phenol, 28,7 g 2-Brompropionsäure-(2-benzyloxy)-ethylester (IV-2) und 14,5 g wasserfreiem Kaliumcarbonat in 150 ml trockenem Acetonitril wurde 4 Stunden unter Rükfluß und Rühren gekocht. Anschließend wurde das Acetonitril unter vermindertem Druck abdestilliert und der Rückstand mit Toluol versetzt. Nach der weiteren Aufarbeitung (analog zu Beispiel 1) wurden 41 g 2-[4-(4-Trifluormethylphenoxy)-phenoxy]-propionsäure-(2-benzyloxy)-ethylester (II-3) erhalten;
$n_D^{20}$: 1,5300

Beispiel 4

nach Verfahren (a)

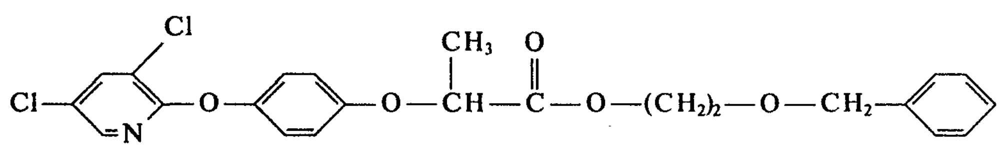
(II-4)

Analog zu Beispiel 1 wurde durch Umsetzung von 4-(3,5-Dichlor-2-pyridyloxy)-phenol-Natriumsalz mit 2-Brompropionsäure-(2-benzyloxy)-ethylester (IV-2) die Verbindung 2-[4-(3,5-Dichlor-2-pyridyloxy)-phenoxy]-propionsäure-(2-benzyloxy)-ethylester (II-4) hergestellt; Schmelzpunkt 59–61°C.

Beispiel 5

nach Verfahren (b)

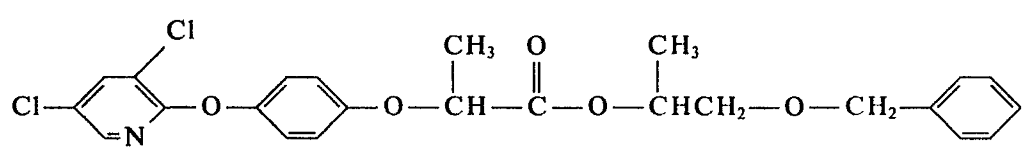
(II-5)

16,6 g 1-Benzyloxy-2-propanol und 10,6 g Triethylamin wurden in 200 ml Toluol gelöst, dann auf 0°C abgekühlt. Zu dieser Lösung wurde bei 0–5°C unter Rühren eine Lösung von 34,7 g 2-[4-(3,5-Dichlor-2-pyridyloxy)-phenoxy]-propionylchlorid in 80 ml Toluol zugetropft. Das Reaktionsgemisch wurde anschließend langsam auf 40–50°C erwärmt und zur Vervollständigung der Umsetzung eine weitere Stunde gerührt. Nach der Aufarbeitung (analog zu Beispiel 1) wurden 43.4 g 2-[4-(3,5-Dichlor-2-pyridyloxy)-phenoxy]-propionsäure-(2-benzyloxy-1-methyl)-ethylester (II-5) in Form eines viskosen Öls erhalten;
$n_D^{20}$: 1,5710.

In analoger Weise können auch die in der folgenden Tabelle 1 aufgeführten Verbindungen der allgemeinen Formel (II) hergestellt werden:

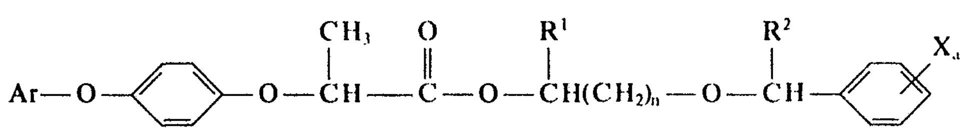
(II)

Tabelle 1

| Verbindung Nr. | Ar | $R^1$ | $R^2$ | n | $X_a$ | Physical. Konstante |
|---|---|---|---|---|---|---|
| II-6 | 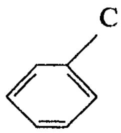 | H | H | 1 | H | $n_D^{20}$ 1,5322 |
| II-7 | 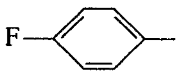 | H | H | 1 | H | $n_D^{20}$ 1,5518 |
| II-8 | 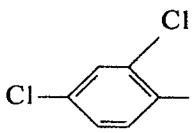 | H | H | 1 | H | $n_D^{20}$ 1,5696 |
| II-9 | 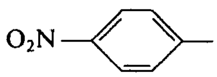 | H | H | 1 | H | $n_D^{20}$ 1,5805 |
| II-10 | 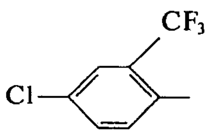 | H | H | 1 | H | $n_D^{20}$ 1,5397 |
| II-11 | 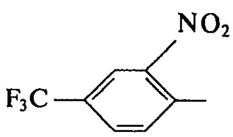 | H | H | 1 | H | $n_D^{20}$ 1,5450 |
| II-12 | 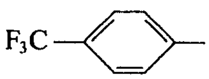 | H | H | 2 | H | $n_D^{20}$ 1,5268 |
| II-13 | 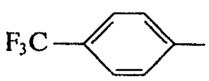 | $-CH_3$ | H | 1 | H | $n_D^{20}$ 1,5260 |
| II-14 | 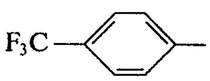 | H | $-CH_3$ | 1 | H | $n_D^{20}$ 1,5266 |
| II-15 | 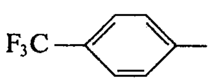 | H | H | 1 | 2-F | $n_D^{20}$ 1,5235 |
| II-16 | 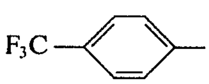 | H | H | 1 | 4-F | $n_D^{20}$ 1,5214 |
| II-17 | 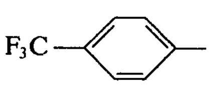 | H | H | 1 | 2-Cl | $n_D^{20}$ 1,5360 |
| II-18 | 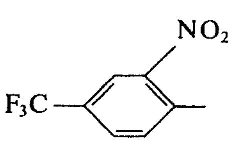 | H | H | 1 | 2-Cl | $n_D^{20}$ 1,5550 |
| II-19 | 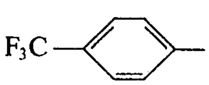 | H | H | 1 | 4-Cl | $n_D^{20}$ 1,5360 |

Fortsetzung

| Verbindung Nr. | Ar | $R^1$ | $R^2$ | n | $X_a$ | Physical. Konstante |
|---|---|---|---|---|---|---|
| II-20 | 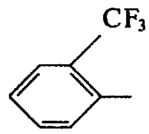 | H | H | 1 | 4-Cl | $n_D^{20}$ 1,5380 |
| II-21 | 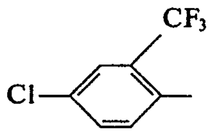 | H | H | 1 | 4-Cl | $n_D^{20}$ 1,5450 |
| II-22 | 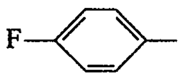 | H | H | 1 | 4-Cl | $n_D^{20}$ 1,5559 |
| II-23 | 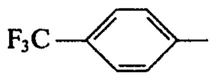 | H | H | 1 | 2,4-$Cl_2$ | $n_D^{20}$ 1,5422 |
| II-24 | 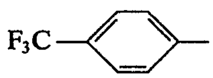 | H | H | 1 | 3,4-$Cl_2$ | $n_D^{20}$ 1,5428 |
| II-25 | 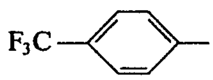 | H | H | 1 | 2-$CH_3$ | $n_D^{20}$ 1,5305 |
| II-26 | 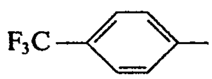 | H | H | 1 | 3-$NO_2$ | $n_D^{20}$ 1,5436 |
| II-27 | 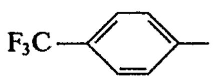 | H | H | 1 | 4-$OCH_3$ | $n_D^{20}$ 1,5343 |
| II-28 | 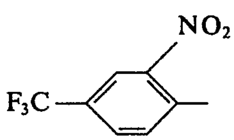 | H | H | 2 | H | $n_D^{20}$ 1,5440 |
| II-29 | 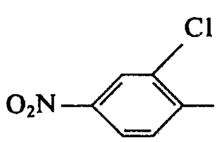 | H | H | 1 | 4-Cl | $n_D^{20}$ 1,5875 |
| II-30 | 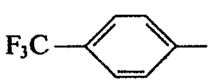 | H | H | 1 | 4-Br | $n_D^{20}$ 1,5450 |
| II-31 | 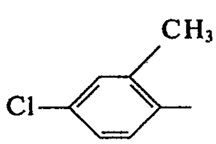 | H | H | 1 | H | $n_D^{20}$ 1,5650 |
| II-32 | 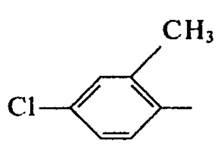 | H | H | 1 | 2-Cl | $n_D^{20}$ 1,5718 |

Fortsetzung

| Verbindung Nr. | Ar | $R^1$ | $R^2$ | n | $X_a$ | Physical. Konstante |
|---|---|---|---|---|---|---|
| II-33 | 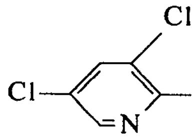 | H | $-CH_3$ | 1 | H | $n_D^{20}$ 1,5715 |
| II-34 | 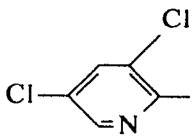 | H | H | 2 | H | $n_D^{20}$ 1,5721 |
| II-35 | 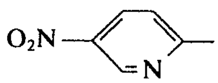 | H | H | 1 | H | Öl |
| II-36 | 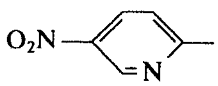 | H | H | 1 | $3\text{-}NO_2$ | Öl |
| II-37 | 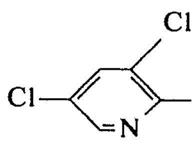 | H | H | 1 | 2-Cl | Fp. 64–66°C |
| II-38 | 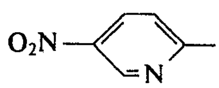 | H | H | 1 | 4-Cl | Öl |
| II-39 | 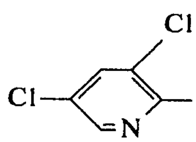 | H | H | 1 | $3\text{-}NO_2$ | $n_D^{20}$ 1,5845 |
| II-40 | 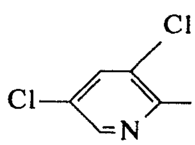 | H | H | 1 | $2\text{-}CH_3$ | Fp. 83–85°C |
| II-41 | 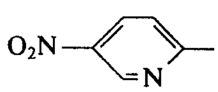 | H | H | 1 | $2,4\text{-}Cl_2$ | Öl |
| II-42 | 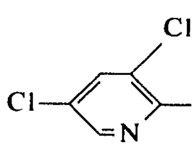 | H | H | 1 | $3,4\text{-}Cl_2$ | $n_D^{20}$ 1,5900 |
| II-43 | 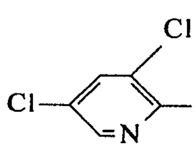 | H | H | 1 | $2,6\text{-}Cl_2$ | $n_D^{20}$ 1,5915 |
| II-44 | 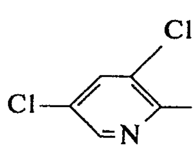 | H | H | 1 | $4\text{-}OCH_3$ | $n_D^{20}$ 1,5735 |

Fortsetzung

| Verbindung Nr. | Ar | $R^1$ | $R^2$ | n | $X_a$ | Physical. Konstante |
|---|---|---|---|---|---|---|
| II-45 | 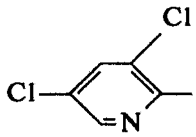 | H | H | 1 | 4-Br | $n_D^{20}$ 1,5870 |
| II-46 | 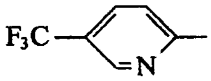 | H | $CH_3$ | 1 | H | $n_D^{20}$ 1,5254 |
| II-47 | 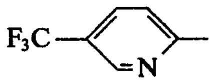 | H | H | 1 | 2-$CH_3$ | $n_D^{20}$ 1,5273 |
| II-48 | 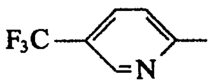 | H | H | 1 | 2-Cl | $n_D^{20}$ 1,5349 |
| II-49 | 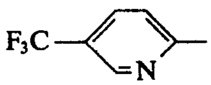 | H | H | 1 | 4-Br | $n_D^{20}$ 1,5440 |
| II-50 | 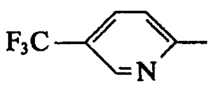 | H | H | 1 | 3-$NO_2$ | $n_D^{20}$ 1,5428 |
| II-51 | 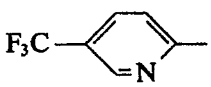 | H | H | 1 | 2,4-$Cl_2$ | $n_D^{20}$ 1,5424 |
| II-52 | 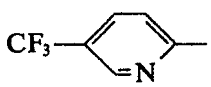 | H | H | 1 | H | Fp. 44–47°C |
| II-53 | 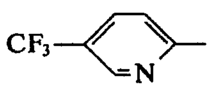 | H | H | 1 | 4-Cl | $n_D^{20}$ 1,5350 |
| II-54 | 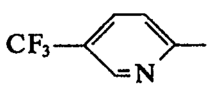 | $CH_3$ | H | 1 | H | $n_D^{20}$ 1,5224 |
| II-55 | 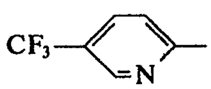 | H | H | 2 | H | $n_D^{20}$ 1,5245 |
| II-56 | 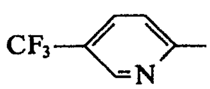 | H | H | 1 | 2-F | Fp. 46–48.5°C |
| II-57 | 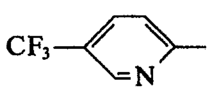 | H | H | 1 | 4-$OCH_3$ | $n_D^{20}$ 1,5335 |

Herstellung von Ausgangsprodukten der allgemeinen Formel (IV):

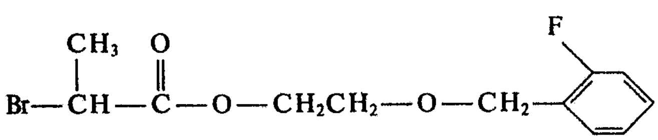

(IV-1)

Eine Lösung von 17,5 g 2-(2-Fluorbenzyloxy)-ethanol und 10,6 g Triethylamin in 150 ml Toluol wurde auf −5°C gekühlt. Unter Rühren wurde bei −5°C bis 0°C eine Lösung von 21,6 g 2-Brompropionylbromid in 30 ml Toluol zugetropft. Das Reaktionsgemisch wurde weitere 2 Stunden bei Raumtemperatur gerührt und anschließend nacheinander mit 1 %iger Natronlauge und Wasser gewaschen. Die Toluol-Lösung wurde dann getrocknet und filtriert. Nach Abdestillieren des Toluols bei vermindertem Druck erhielt man 28,2 g Brompropionsäure-[2-(2-fluorbenzyloxy)]-ethylester (IV-1);
$n_D^{20}$: 1,5038

Auf analoge Weise wurden die in der folgenden Tabelle 2 aufgeführten Verbindungen der allgemeinen Formel (IV) hergestellt:

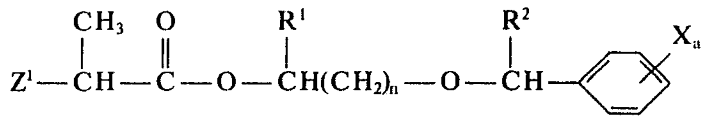

(IV)

Tabelle 2

| Verbindung Nr. | $Z^1$ | $R^1$ | $R^2$ | n | $X_a$ | Physical. Konstante |
|---|---|---|---|---|---|---|
| IV-2 | Br | H | H | 1 | H | $n_D^{20,5}$ 1,5182 |
| IV-3 | Cl | H | H | 1 | 4-F | $n_D^{20}$ 1,5004 |
| IV-4 | Br | H | H | 1 | 4-F | $n_D^{20}$ 1,5040 |
| IV-5 | Br | H | H | 1 | 2-Cl | $n_D^{20,5}$ 1,5302 |
| IV-6 | Br | H | H | 1 | 4-Cl | $n_D^{20}$ 1,5289 |
| IV-7 | Cl | H | H | 1 | 4-Br | $n_D^{20,5}$ 1,5321 |
| IV-8 | Br | H | H | 1 | 4-Br | $n_D^{20,5}$ 1,5434 |
| IV-9 | Br | H | H | 1 | 2-$CH_3$ | $n_D^{20}$ 1,5186 |
| IV-10 | Br | H | H | 1 | 2-$OCH_3$ | $n_D^{20,5}$ 1,5260 |
| IV-11 | Br | H | H | 1 | 3-$NO_2$ | $n_D^{20}$ 1,5394 |
| IV-12 | Br | H | H | 1 | 2,4-$Cl_2$ | $n_D^{20}$ 1,5395 |
| IV-13 | Br | H | H | 1 | 3,4-$Cl_2$ | $n_D^{20}$ 1,5409 |
| IV-14 | Cl | H | H | 1 | 2,6-$Cl_2$ | $n_D^{20}$ 1,5412 |
| IV-15 | Br | $CH_3$ | H | 1 | H | $n_D^{20,5}$ 1,5100 |
| IV-16 | Br | H | $CH_3$ | 1 | H | $n_D^{20,5}$ 1,5116 |
| IV-17 | Br | H | H | 2 | H | $n_D^{20,5}$ 1,5130 |
| IV-18 | Br | H | H | 1 | 4-$OCH_3$ | $n_D^{20,5}$ 1,5250 |

**Patentansprüche:**

1. Selektiv-herbizide Mittel, gekennzeichnet durch einen Gehalt an einer Wirkstoffkombination bestehend aus

(1) 4-Amino-3-methyl-6-phenyl-1,2,4-triazin-5(4 H)-on (Metamitron) der Formel I

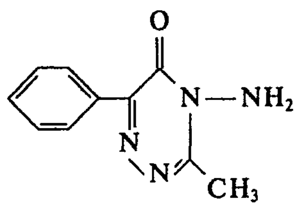

(I)

und

(2) mindestens einem substituierten Phenoxypropionsäureester der allgemeinen Formel II

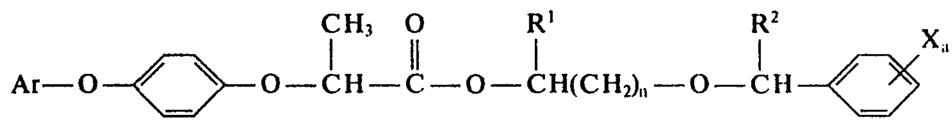

(II)

worin

$R^1$ für Wasserstoff oder Methyl steht,
$R^2$ für Wasserstoff oder Methyl steht,
n für 1 oder 2 steht,
X für Wasserstoff, Methyl, Methoxy, Halogen und/oder Nitro steht,
a für 1 oder 2 steht,
Ar für den Rest

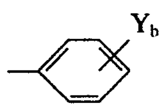 oder 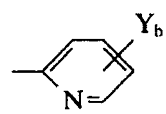

steht,
Y für $C_{1-4}$-Alkyl, Halogen, Trifluormethyl, Nitro und/oder Cyano steht und
b für 1, 2 oder 3 steht,

2. Selektiv-herbizide Mittel gemäß Anspruch 1, dadurch gekennzeichnet, daß in den Wirkstoffkombinationen das Gewichtsverhältnis von Wirkstoff der Formel (I) zu dem Wirkstoff aus der Gruppe (II) zwischen 1 : 0,001 und 1 : 20, vorzugsweise 1 : 0,01 und 1 : 10 liegt.

3. Verfahren zur selektiven Bekämpfung von Unkraut in Rübenkulturen, dadurch gekennzeichnet, daß man eine Wirkstoffkombination gemäß Anspruch 1 vor oder nach dem Auflaufen der Pflanzen auf die Rübenfelder einwirken läßt.

4. Verwendung von Wirkstoffkombinationen gemäß Anspruch 1 zur selektiven Bekämpfung von Unkraut in Rübenkulturen.

5. Verfahren zur Herstellung von selektiv-herbiziden Mitteln, dadurch gekennzeichnet, daß man eine Wirkstoffkombination gemäß Anspruch 1 mit Streckmitteln und/oder oberflächenaktiven Mitteln vermischt.

6. Selektiv-herbizide Mittel gemäß Anspruch 1, dadurch gekennzeichnet, daß die Wirkstoffkombination aus Metamitron (I) und der Verbindung der Formel II-1

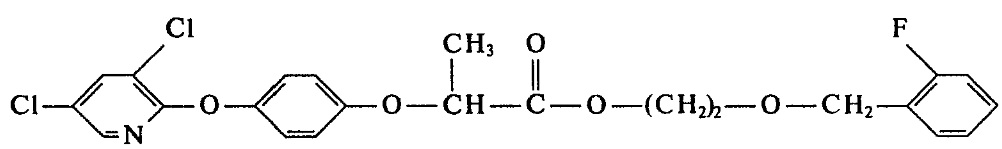

(II-1)

besteht.

7. Selektiv-herbizide Mittel gemäß Anspruch 1, dadurch gekennzeichnet, daß die Wirkstoffkombination aus Metamitron (I) und der Verbindung der Formel II-2

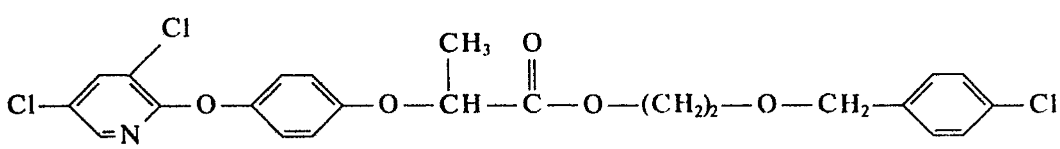

(II-2)

besteht.

8. Selektiv-herbizide Mittel gemäß Anspruch 1, dadurch gekennzeichnet, daß die Wirkstoffkombination aus Metamitron (I) und der Verbindung der Formel II-3

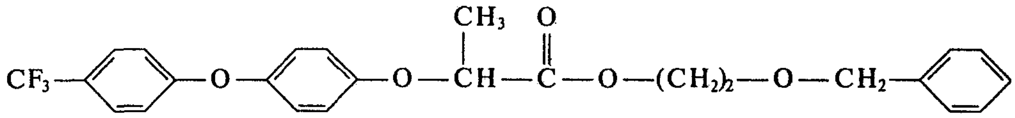

(II-3)

besteht.

9. Selektiv- herbizide Mittel gemäß Anspruch 1, dadurch gekennzeichnet, daß die Wirkstoffkombination aus Metamitron (I) und der Verbindung der Formel II-4

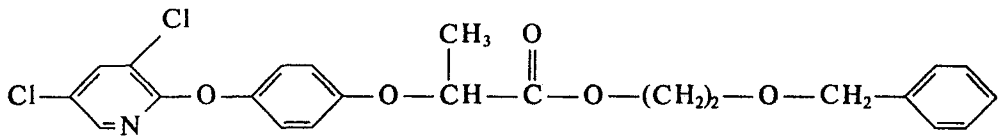

(II-4)

besteht.

10. Selektiv-herbizide Mittel gemäß Anspruch 1, dadurch gekennzeichnet, daß die Wirkstoffkombination aus Metamitron (I) und der Verbindung der Formel II-5

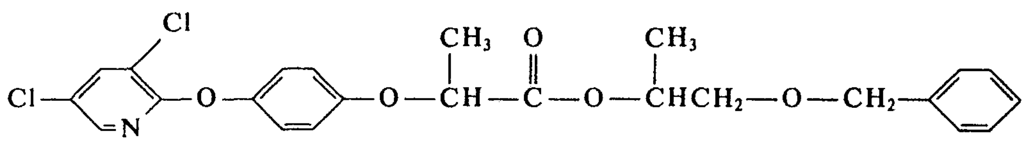

(II-5)

besteht.

**Claims:**

1. Selective herbicidal agents, characterised in that they contain an active compound combination consisting of

(1) 4-amino-3-methyl-6-phenyl-1,2,4-triazin-5(4 H)-one (metamitron) of the formula I

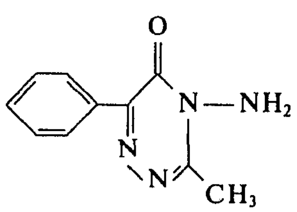

(I)

and

(2) at least one substituted phenoxypropionic acid ester of the general formula II

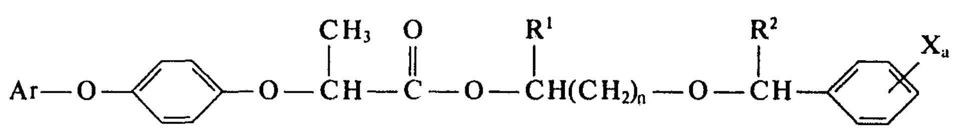

(II)

wherein

$R^1$ represents hydrogen or methyl,
$R^2$ represents hydrogen or methyl,
n represents 1 or 2,
X represents hydrogen, methyl, methoxy, halogen and/or nitro,
a represents 1 or 2,
Ar represents the radical

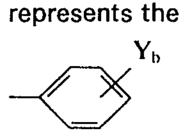

ou

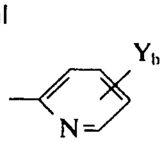

Y represents $C_{1-4}$-alkyl, halogen, trifluoromethyl, nitro and/or cyano and
b represents 1, 2 or 3.

2. Selective herbicidal agents according to Claim 1, characterised in that, in the active compound combinations, the weight ratio of the active compound of the formula (I) to the active compound from the group (II) is between 1 : 0.001 and 1 : 20, preferably between 1 : 0.01 and 1 : 10.

3. Method of selectively combating weeds in beet crops, characterised in that an active compound combination according to Claim 1 is allowed to act on the beet fields before or after the emergence of the plants.

4. Use of active compound combinations according to Claim 1 for selectively combating weeds in beet crops.

5. Process for the preparation of selective herbicidal agents, characterised in that an active compound combination according to Claim 1 is mixed with extenders and/or surface-active agents.

6. Selective herbicidal agents according to Claim 1, characterised in that the active compound combination consists of metamitron (I) and the compound of the formula II-1

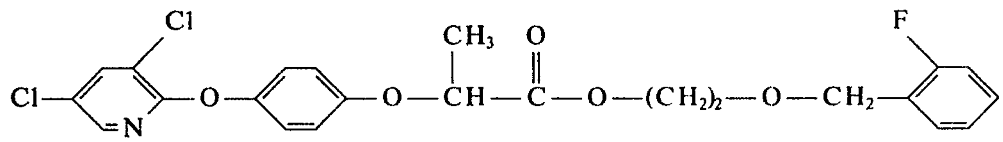

(II-1)

7. Selective herbicidal agents according to Claim 1, characterised in that the active compound combination consists ot metamitron (I) and the compound of the formula II-2

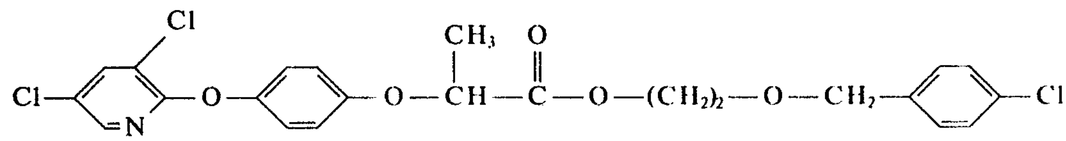

(II-2)

8. Selective herbicidal agents according to Claim 1, characterised in that the active compound combination consists of metamitron (I) and the compound of the formula II-3

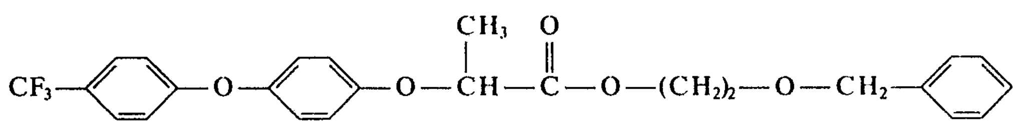

(II-3)

9. Selective herbicidal agents according to Claim 1, characterised in that the active compound combination consists of metamitron (I) and the compound of the formula II-4

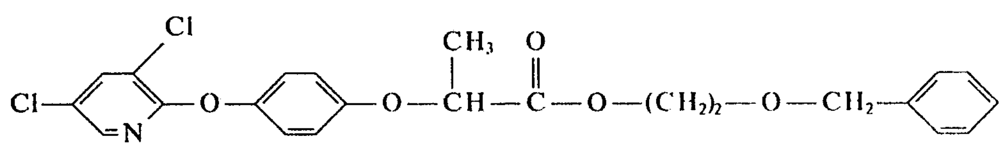

(II-4)

10. Selective herbicidal agents according to Claim 1, characterised in that the active compound combination consists of metamitron (I) and the compound of the formula II-5

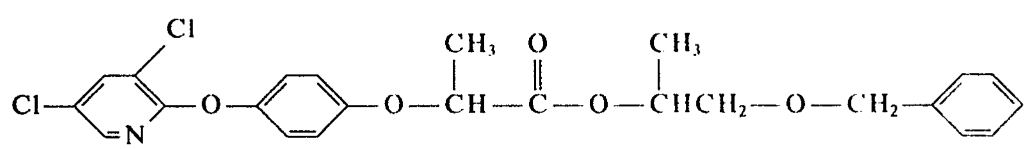

(II-5)

**Revendications:**

1. Compositions herbicides sélectives, caractérisées par une teneur en une association de substances actives comprenant

(1) De la 4-amino-3-méthyl-6-phényl-1,2,4-triazine-5(4 H)-one (métamitron) de formule I

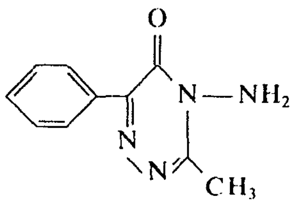

(I)

et

(2) au moins un ester d'acide phénoxypropionique substitué de formule générale II

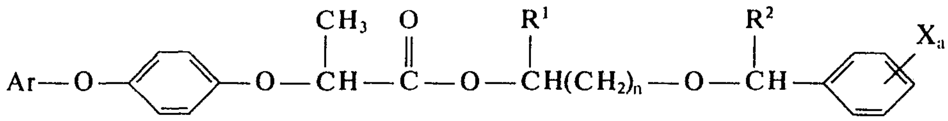

(II)

dans laquelle

$R^1$ représente l'hydrogène ou le groupe méthyle,
$R^2$ représente l'hydrogène ou le groupe méthyle,
n a la valeur 1 ou 2,
X représente l'hydrogène, un groupe méthyle, méthoxy, un halogène et/ou un groupe nitro,
a a la valeur 1 ou 2,
Ar représente le reste

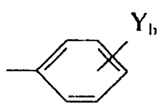 ou 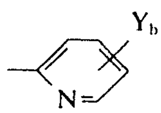

Y est un groupe alkyle en $C_1$ à $C_4$, un halogène, un groupe trifluorométhyle, nitro et/ou cyano et
b a la valeur 1, 2 ou 3.

2. Compositions herbicides sélectives suivant la revendication 1, caractérisées en ce que le rapport en poids de la substance active de formule (I) à la substance active du groupe (II) dans les associations de substances actives se situe entre 1 : 0,001 et 1 : 20, de préférence entre 1 : 0,01 et 1 : 10.

3. Procédé de lutte sélective contre des mauvaises herbes dans des cultures de betteraves, caractérisé en ce qu'on fait agir une association de substances actives suivant la revendication 1, avant ou après la levée des plantes sur les champs de betteraves.

4. Utilisation d'associations de substances actives suivant la revendication 1 pour la lutte sélective contre des mauvaises herbes dans des cultures de betteraves.

5. Procédé de production de compositions herbicides sélectives, caractérisé en ce qu'on mélange une association de substances actives suivant la revendication 1 avec des diluants et/ou des agents tensioactifs.

6. Compositions herbicides sélectives suivant la revendication 1, caractérisées en ce que l'association de substances actives est formée de métamitron (I) et du composé de formule II-1

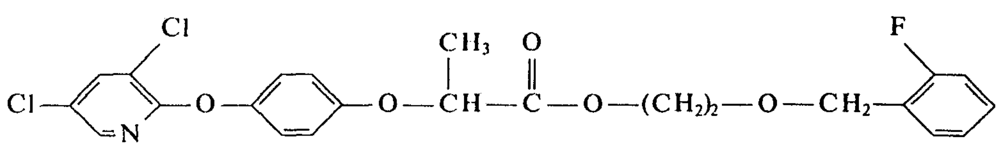

(II-1)

7. Compositions herbicides sélectives suivant la revendication 1, caractérisées en ce que l'association de substances actives comprend du métamitron (I) et le composé de formule II-2

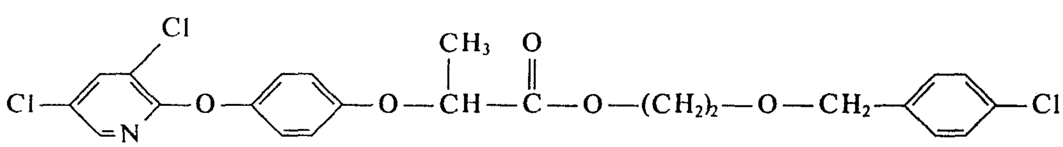

(II-2)

8. Compositions herbicides sélectives suivant la revendication 1, caractérisées en ce que l'association de substances actives est formée de métamitron (I) et du composé de formule II-3

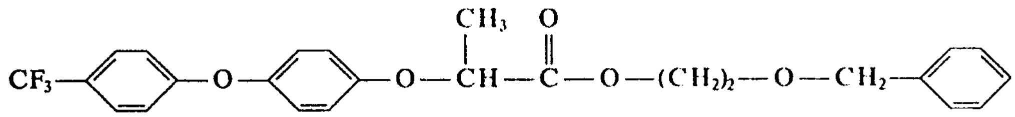 (II-3)

9. Compositions herbicides sélectives suivant la revendication 1, caractérisées en ce que l'association de substances actives est formée de métamitron (I) et du composé de formule II-4

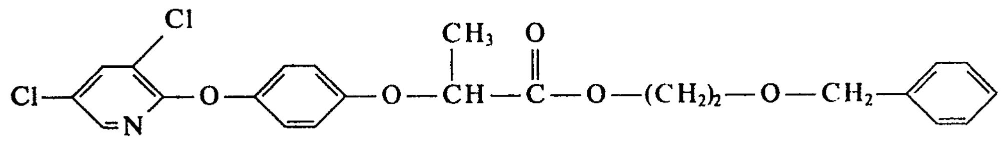 (II-4)

10. Compositions herbicides sélectives suivant la revendication 1, caractérisées en ce que l'association de substances actives est formée de métamitron (I) et du composé de formule II-5

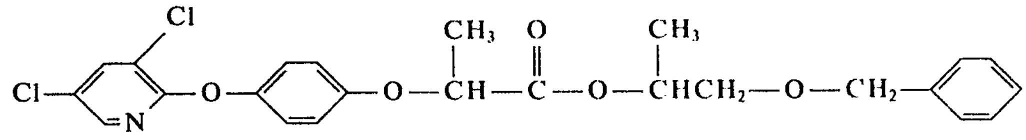 (II-5)